(19) Europäisches Patentamt
European Patent Office
Office européen des brevets

(11) Veröffentlichungsnummer: **0 229 011**
**A1**

(12) **EUROPÄISCHE PATENTANMELDUNG**

(21) Anmeldenummer: **87100012.1**

(22) Anmeldetag: **02.01.87**

(51) Int. Cl.⁴: **C 07 D 249/04**
A 61 K 31/41

(30) Priorität: **06.01.86 CH 5/86**

(43) Veröffentlichungstag der Anmeldung:
**15.07.87 Patentblatt 87/29**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Meier, René**
**Rickenbacherstrasse 13**
**CH-4463 Buus (CH)**

(74) Vertreter: **Zumstein, Fritz, Dr. et al**
**Bräuhausstrasse 4**
**D-8000 München 2 (DE)**

(54) **Trisubstituierte Triazole.**

(57) Trisubstituierte 1,2,3-Triazole der Formel

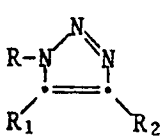

(I),

worin R einen aliphatischen Rest bedeutet, $R_1$ Amino, Carbamoyl, $C_1$-$C_7$-Alkylcarbamoyl, Di-$C_1$-$C_7$-alkyl-carbamoyl oder $C_1$-$C_7$-Alkyl bedeutet und $R_2$ Carbamoyl, $C_1$-$C_7$-Alkylcarbamoyl oder Di-$C_1$-$C_7$-alkylcarbamoyl bedeutet, können als Arzneimittelwirkstoffe verwendet werden und werden in an sich bekannter Weise hergestellt.

EP 0 229 011 A1

**Beschreibung**

## Trisubstituierte Triazole

Die Erfindung betrifft trisubstituierte 1,2,3-Triazole der Formel

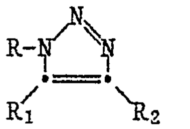

(I),

worin R einen aliphatischen Rest bedeutet, $R_1$ Amino, $C_1$-$C_4$-Alkylamino, Di-$C_1$-$C_4$-Alkylamino, Carbamoyl, $C_1$-$C_7$-Alkylcarbamoyl, Di-$C_1$-$C_7$-alkylcarbamoyl oder $C_1$-$C_7$-Alkyl bedeutet und $R_2$ Carbamoyl, $C_1$-$C_7$-Alkylcarbamoyl oder Di-$C_1$-$C_7$-carbamoyl bedeutet, ihre Herstellung und Verwendung, pharmazeutische Präparate, enthaltend eine Verbindung der Formel I, und deren Herstellung.

Ein aliphatischer Rest R steht insbesondere für $C_1$-$C_{10}$-Alkyl oder $C_3$-$C_{10}$-Alkenyl, welches vorzugsweise eine oder zwei, in erster Linie isolierte, Doppelbindungen aufweist. Derartige Reste sind in erster Linie über ein sekundäres C-Atom gebunden. $C_3$-$C_{10}$-Alkenyl weist die Doppelbindung vorzugsweise in höherer als der 1-Stellung auf.

Die vor- und nachstehend verwendeten Allgemeindefinitionen haben, sofern nicht abweichend definiert, in erster Linie die folgenden Bedeutungen: $C_1$-$C_4$-Alkylamino ist z.B. Methyl-, Ethyl-, Propyl- oder Butylamino.

Di-$C_1$-$C_4$-Alkylamino ist z.B. Dimethylamino, N-Methyl-N-ethylamino, Diäthylamino oder N-Methyl-N-propyl-amino.

$C_1$-$C_7$-Alkylcarbamoyl ist vorzugsweise $C_1$-$C_4$-Alkylcarbamoyl, z.B. N-Methyl-, N-Ethyl-, N-Propyl-, N-Isopropyl oder N-Butylcarbamoyl, kann aber auch N-Isobutyl-, N-sek.Butyl- oder N-tert.Butylcarbamoyl oder eine N-Pentyl-, N-Hexyl- oder N-Heptylcarbamoylgruppe sein.

Di-$C_1$-$C_7$-Alkylcarbamoyl ist vorzugsweise Di-$C_1$-$C_4$-Alkylcarbamoyl, z.B. N,N-Dimethyl-, N,N, Diethyl-, N-Methyl-N-ethyl- oder N-Methyl-N-propylcarbamoyl.

$C_1$-$C_7$-Alkyl R ist vorzugsweise $C_1$-$C_4$-Alkyl, z.B. Methyl, Ethyl, Propyl, Isopropyl, Butyl, Isobutyl, sek.Butyl oder Tert.Butyl, kann aber auch eine Pentyl-, Hexyl- oder Heptylgruppe sein.

$C_1$-$C_{10}$-Alkyl R ist z.B. Methyl, Ethyl, n-Propyl, Isopropyl, n-Butyl, Isobutyl, sek-Butyl, tert-Butyl, ein entsprechender Pentyl-, Hexyl-, Heptyl-, Octyl-, Nonyl- oder Decylrest, insbesondere jedoch ein über ein sekundäres C-Atom gebundener $C_5$-$C_{10}$-Alkylrest, wie 1-Ethyl-propyl (3-Pentyl), 1-Ethyl-n-butyl (3-Hexyl), 1-n-Propyl-n-butyl (4-Heptyl), 1-n-Propyl-n-pentyl (4-Octyl), 1-n-Butyl-n-pentyl (5-Nonyl) oder 1-n-Butyl-n-hexyl (5-Decyl).

$C_3$-$C_{10}$-Alkenyl R weist insbesondere eine oder zwei Doppelbindungen auf und ist z.B. 1-Propenyl, Allyl, Methallyl, 1-, 2-, 3-Butenyl, 1,3-Butadienyl, 1-, 2-, 3-, 4-Pentenyl, 1,3-, 2,4-, 1,4-Pentadienyl, ein entsprechender Hexenyl-, Heptenyl-, Octenyl-, Nonenyl- oder Decenylrest, insbesondere über ein sekundäres C-Atom gebundene $C_5$-$C_{10}$-Alkenylreste, wie 1-Ethyl-allyl (3-Pent-1-enyl), 1-Vinyl-allyl (3-Penta-1,4-dienyl), 1-Ethyl-2- oder 1-Ethyl-3-butenyl (4-Hex-2-enyl bzw. -1-yl), 1-n-Propyl-allyl (3-Hex-1-enyl), 1-Allyl-2-oder 1-Allyl-3-butenyl (4-Hepta-2,6-dienyl bzw. -1,6-dienyl), 1-n-Propyl-2- oder 1-n-Propyl-3-butenyl (4-Hept-2-enyl- bzw. 1-enyl), ferner 2-Butenyl-3-pentenyl (5-Nona-2,7-dienyl) oder 3-Butenyl-4-pentenyl (5-Nona-1,8-dienyl).

Die Erfindung betrifft vorzugsweise solche Verbindungen der Formel I, worin $R_1$ Amino oder in zweiter Linie $C_1$-$C_4$-Alkyl- oder Di-$C_1$-$C_4$-Alkylamino und $R_2$ Carbamoyl ist.

Die neuen Verbindungen besitzen wertvolle pharmakologische Eigenschaften, insbesondere eine ausgeprägte antikonvulsive Wirksamkeit mit neuartigem Wirkungsspektrum. Die ausgezeichnete antikonvulsive Wirksamkeit der erfindungsgemäss bereitgestellten Verbindungen zeigt sich insbesondere an Primaten. So bewirkt 5-Amino-1-(4-Heptyl)-1H-1,2,3-triazol-4-carboxamid in Tagesdosen von 50 mg/kg p.o. über die gesamte 15-tägige Behandlungsdauer eine starke Abnahme sowohl der Anzahl als auch der Intensität der durch Aluminiumoxidimplantation erzeugten fokalen Konvulsionen des Rhesusaffen. Das neuartige Wirkungsspektrum der erfindungsgemässen Verbindungen kann beispielsweise durch ihr Verhalten in den nachstehenden pharmakologischen und biochemischen Epilepsiemodellen charakterisiert werden:

1) Schutzwirkung gegen Elektroschock-induzierte Konvulsionen (Maus, Ratte): deutliche Aktivität in Dosen ab etwa 30 mg/kg p.o., für z.B. 5-Amino-1-(4-hexyl)-1M-1,2,3-triazol-4-carboxamid $ED_{50}$ = 100 mg/kg p.o. (Maus) bzw. 50 mg/kg p.o. (Ratte);

2) Metrazol-Antagonismus an der Maus: deutliche Aktivität in Dosen von etwa 30 bis 600 mg/kg p.o., für z.B. 5-Amino-1-(4-hexyl)-1H-1,2,3-triazol-4-carboxamid $ED_{50}$ = 210 mg/kg p.o.;

3) Schutzwirkung gegen Picrotoxin-induzierte Konvulsionen (Maus): keine oder nur sehr schwache Aktivität, für z.B. 5-Amino-1-(4-hexyl)-1H-1,2,3-triazol-4-carboxamid in Dosen bis einschliesslich 300 mg/kg p.o. keine Wirkung feststellbar;

4) Schutzwirkung gegen experimentellen Strychninkrampf (Maus): deutliche Aktivität, für z.B. 5-Amino-1-(4-hexyl)-1H-1,2,3-triazol-4-carboxamid $ED_{50}$ = 300 mg/kg p.o.;

5) Einfluss auf die $\gamma$-Aminobuttersäure-(GABA)-Umsatzgeschwindigkeit und Konzentration an Nervenfaserenden (Mäusehirn): dosisabhängige Reduktion der GABA-Umsatzgeschwindigkeit, dabei bleibt die GABA-Konzentration unverändert, d.h. Wirkung auf die GABA-Neuro-transmission und kein nachweisbarer Einfluss auf den GABA-Kata-bolismus;

6) Kindling-Epilepsiemodell (Ratte): die Entwicklung sowohl der Nachentladung als auch

der Symptomatologie wird stark verlangsamt.

Auf Grund der vorstehenden Befunde, insbesondere in den biochemischen Modelle 5) und 6), wird angenommen, dass die erfindungsgemäss bereitgestellten Verbindungen eine ausgeprägte günstige Wirkung auf die GABA-Neurotransmission besitzen, ohne den GABA-Katabolismus nennenswert zu beeinflussen. Deswegen und wegen ihrer ausgezeichneten antikonvulsiven Wirkung an Primaten und Nagetieren werden die Verbindungen der Formel I als zur Behandlung verschiedenartigster Epilepsietypen, insbesondere des fokalen Formenkreises, deren Therapie bislang noch als besonders problematisch gilt, als vorzüglich geeignet angesehen. Sie können dementsprechend als antikonvulsive, beispielsweise antiepileptische, Arzneimittelwirkstoffe verwendet werden.

Die Erfindung betrifft in erster Linie Verbindungen der Formel I, worin R über ein sekundäres C-Atom gebundenes $C_5$-$C_{10}$-Alkyl bzw. $C_5$-$C_{10}$-Alkenyl, welches eine oder zwei Doppelbindungen aufweist, bedeutet, $R_1$-Amino, $C_1$-$C_4$-Alkylamino, Di-$C_1$-$C_4$-Alkylamino, Carbamoyl, $C_1$-$C_4$-Alkylcarbamoyl, Di-$C_1$-$C_4$-alkyl-carbamoyl oder $C_1$-$C_4$-Alkyl bedeutet und $R_2$-Carbamoyl, $C_1$-$C_4$-Alkylcarbamoyl oder Di-$C_1$-$C_4$-alkyl-carbamoyl bedeutet, vorzugsweise solche, in denen $R_1$ Amino oder in zweiter Linie $C_1$-$C_4$-Alkyl- oder Di-$C_1$-$C_4$-Alkylamino und $R_2$ Carbamoyl ist.

Die Erfindung betrifft vor allem Verbindungen der Formel, worin R über ein sekundäres C-Atom gebundenes $C_5$-$C_8$-Alkyl bzw. $C_5$-$C_8$-Alkenyl mit einer oder zwei Doppelbindungen bedeutet, $R_1$ Amino, Carbamoyl oder $C_1$-$C_4$-Alkyl bedeutet und $R_2$ Carbamoyl, $C_1$-$C_4$-Alkylcarbamoyl oder Di-$C_1$-$C_4$-alkyl-carbamoyl bedeutet, vorzugsweise solche, in denen $R_1$ Amino und $R_2$ Carbamoyl ist.

Die Erfindung betrifft in erster Linie Verbindungen der Formel I, worin R 1-n-Propyl-n-butyl oder 1-Allyl-3-butenyl bedeutet, $R_1$ Amino, Carbamoyl oder $C_1$-$C_4$-Alkyl, insbesondere Methyl, bedeutet und $R_2$ Carbamoyl ist, vorzugsweise solche, in denen $R_1$ Amino und $R_2$ Carbamoyl ist.

Die Erfindung betrifft in allererster Linie Verbindungen der Formel I, worin R 1-n-Propyl-n-butyl bedeutet, $R_1$ Amino, Carbamoyl oder Methyl bedeutet und $R_2$ Carbamoyl ist, vorzugsweise diejenige, in der $R_1$ Amino ist.

Die Erfindung betrifft insbesondere die in den Beispielen genannten neuen Verbindungen und Verfahren zu ihrer Herstellung.

Ebenfalls Gegenstand der Erfindung sind Verfahren zur Herstellung der erfindungsgemässen Verbindungen. Die Herstellung von Verbindungen der Formel I erfolgt in an sich bekannter Weise und ist z.B. dadurch gekennzeichnet, dass man

a) eine Verbindung der Formel R-$N_3$ (IIa) mit einer Verbindung der Formel

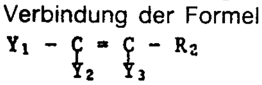

(IIb)

worin $Y_1$ Di-$C_1$-$C_4$-Alkylamino, Carbamoyl, $C_1$-$C_7$-Alkylcarbamoyl, $D_1$-$C_1$-$C_7$-Alkylcarbamoyl oder $C_1$-$C_7$-Alkyl bedeutet und $Y_2$ Hydroxy und $Y_3$ Wasserstoff bedeutet oder $Y_2$ und $Y_3$ gemeinsam eine zusätzliche Bindung darstellen, oder worin $Y_1$ und $Y_2$ gemeinsam Imino darstellen und $Y_3$ Wasserstoff ist, oder einem Tautomeren und/oder Salz davon umsetzt oder

b) in einer Verbindung der Formel

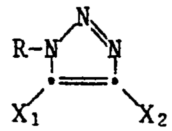

(III)

oder einem Salz davon, worin $X_1$ einen in $R_1$ überführbaren Rest bedeutet und $X_2$ einen in $R_2$ überführbaren Rest oder $R_2$ bedeutet oder worin $X_1$ für $R_1$ steht und $X_2$ einen in $R_2$ überführbaren Rest bedeutet, $X_1$ und/oder $X_2$ in $R_1$ überführt und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt.

Tautomere von Verbindungen der Formel IIb sind beispielsweise Ketotautomere der Formel $Y_1$-C(=O)-$CH_2$-$R_2$ (IIc) von Enolen (IIa; $Y_2$ = Hydroxy, $Y_3$ = Wasserstoff) bzw. zu Keteniminen (IIa; $Y_1+Y_2$ = Imino, $Y_3$ = Wasserstoff) tautomere Nitrile der Formel N≡C-$CH_2$-$R_2$ (IId).

Salze von Verbindungen IIb bzw. ihrer Tautomeren sind beispielsweise Metall-, wie Alkalimetall, z.B. Natrium- oder Kaliumsalze derselben.

Ein in $R_1$ überführbarer Rest $X_1$ ist beispielsweise durch Solvolyse in Carbamoyl, $C_1$-$C_7$-Alkylcarbamoyl oder Di-$C_1$-$C_7$-alkylcarbamoyl $R_1$ überführbares gegebenenfalls funktionell abgewandeltes Carboxy oder geschütztes Amino bzw. $C_1$-$C_4$-Alkylamino, während als in $R_2$ überführbarer Rest $X_2$ beispielsweise durch Solvolyse in Carbamoyl, $C_1$-$C_7$-Alkylcarbamoyl oder Di-$C_1$-$C_7$-alkylcarbamoyl überführbares ggf. funktionell abgewandeltes Carboxy in Frage kommt. Derartiges funktionell abgwandeltes Carboxy ist z.B. verestertes Carboxy, wie $C_1$-$C_7$-Alkoxycarbonyl, Cyano, anhydridisiertes Carboxy, wie Halogen-carbonyl, $C_1$-$C_7$-Alkanoyloxycarbonyl oder $C_1$-$C_4$-Alkoxy-carbonyl-oxycarbonyl, oder eine $C_1$-$C_4$-Alkoxy-iminomethylgruppe der Formel -C(=NH)-$C_1$-$C_7$-Alkoxy.

Geschütztes Amino $X_1$ bedeutet beispielsweise leicht spaltbares Acylamino, Arylmethylamino, 2-$C_3$-$C_7$-Alkenylamino, 1-Acyl-alk-1-enyl-amino, Silylamino oder Azido. Unter Acylamino ist beispielsweise gegebenenfalls, z.B. durch Halogen oder Aryl, substituiertes $C_1$-$C_7$-Alkanoylamino, wie Formyl-, Acetyl- oder Propionyl-amino, 2-Chlor-, 2-Brom-, 2,2,2-Trifluor- oder 2,2,2-Trichloracetylamino, gegebenenfalls, z.B. durch Halogen, $C_1$-$C_7$-Alkoxy oder Nitro, substituiertes Benzoylamino, wie 4-Chlor-, 4-Methoxy- oder 4-Nitrobenzoylamino, $C_1$-$C_7$-Alkoxycarbonylamino, wie tert-Butyloxycarbonylamino, durch gegebenenfalls z.B. $C_1$-$C_7$-Alkyl, $C_1$-$C_7$-Alkoxy, Hydroxy, Halogen und/oder Nitro aufweisendes Aryl substituiertes Methoxycarbonylamino, wie gegebenenfalls entsprechend substituiertes Benzyloxycarbonyl-, Di- oder Triphenylmethoxycarbonylamino [z.B. 4-Nitrobenzyloxycarbonyl-, Benzhydryloxycarbonyl- oder Bis-(4-methoxyphenyl)-methoxycarbonyl-amino], Aroylmethoxycarbonylamino, wie gegebenenfalls, z.B. durch Halogen, substituiertes Benzoyloxycarbonylamino [z.B. Phenacyloxycarbonylamino], Halogen-$C_2$-$C_7$-alkoxycarbonylamino, wie 2,2,2-Trichlor-, 2-Brom- oder 2-Iod-ethoxycarbonylamino, oder 2-(trisubstituiertes Silyl)-ethoxycarbonylamino, wie 2-Tri-$C_1$-$C_7$-alkylsilylethoxycarbonylamino oder 2-Triarylsilyl-ethoxycarbonylamino [z.B. 2-Trimethylsilyl- oder Triphenylsilylethoxycarbonylamino], zu verstehen. Als Arylmethylamino kommt z.B. Mono-, Di- oder Triphenylmethylamino, und als 2-$C_3$-$C_7$-Alkenylamino z.B. Allylamino in Betracht. 1-Acyl-prop-1-enylamino stellt z.B. 1-$C_1$-$C_7$-Alkanoyl-prop-1-en-2-ylamino, gegebenenfalls, z.B. durch $C_1$-$C_7$-Alkyl, $C_1$-$C_7$-Alkoxy, Halogen oder Nitro, substituiertes 1-Benzoyl-prop-1-en-2-ylamino oder 1-$C_1$-$C_7$-Alkoxycarbonyl-prop-1-en-2-ylamino, wie 1-Acetyl-prop-1-en-2-yl-oder 1-Ethoxycarbonyl-prop-1-en-2-ylamino, dar. Entsprechendes gilt für geschütztes $C_1$-$C_4$-Alkylamino $X_1$.

Bevorzugtes geschütztes Amino ist z.B. $C_1$-$C_7$-Alkanoylamino, wie Formyl- oder Acetylamino.

Das Ausgangsmaterial der Formel III, worin $X_1$ bzw. $X_2$ z.B. für eine $C_1$-$C_7$-Alkoxy-iminomethylgruppe steht, kann z.B. in Form von Säureadditionssalzen mit Säuren, vorliegen, während Ausgangsverbindungen mit sauren Gruppen ($X_1$ oder $X_2$ = CO-OH), Salze mit Basen bilden können. Entsprechende Säuren sind beispielsweise starke anorganische Säuren, wie Mineralsäuren, z.B. Schwefelsäure, Phosphorsäure oder Halogenwasserstoffsäuren, mit starken organischen Carbonsäuren, wie $C_1$-$C_7$-Alkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder mit Sulfonsäuren, wie Niederalkan- oder gegebenenfalls substituierte Benzolsulfonsäure, z.B. Methan- oder p-Toluolsulfonsäure. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di- bzw. Trihydroxy-$C_1$-$C_7$-alkylamine, Hydroxy-$C_1$-$C_7$-alkyl-$C_1$-$C_7$-alkyl-amine oder wie Polyhydroxy-$C_1$-$C_7$-alkylamine. Cyclische Amine sind z.B. Morpholin, Thiomorpholin, Piperidin oder Pyrrolidin. Als Mono-$C_1$-$C_7$-alkylamine kommen beispielsweise Ethyl- oder tert.-Butylamin, als Di-$C_1$-$C_7$-alkylamine beispielsweise Diethyl- oder Diisopropylamin, als Tri-$C_1$-$C_7$-alkylamine beispielsweise Trimethyl- oder Triethylamin in Betracht. Entsprechende Hydroxy-$C_1$-$C_7$-alkylamine sind z.B. Mono-, Di- bzw. Triethanolamine, und Hydroxy-$C_1$-$C_7$-alkylamine sind z.B. N,N-Dimethylamino- oder N,N-Diethylaminoethanol, ferner Glucosamin als Polyhydroxy-$C_1$-$C_7$-alkylamin.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -10° bis zur Siedetemperatur des Reaktionsmediums, vorzugsweise von etwa -10° bis etwa 150°C, und, falls erforderlich, in einem geschlossenen Gefäss, unter Druck, in einer Inertgasatmosphäre und/oder unter wasserfreien Bedingungen arbeitet.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalin-amine, $C_1$-$C_7$-Alkylamine, basische Heterocyclen, Ammoniumhydroxide sowie carbocyclische Amine in Frage. Beispielhaft seien Lithium-hydroxid, Natrium-hydroxid, -hydrid, -amid, -ethylat, Kalium-tert-butylat, -carbonat, Lithiumtriphenylmethylid, -diisopropylamid, Kalium-3-(aminopropyl)-amid, -bis-(trimethylsilyl)-amid, Dimethyl-aminonaphthalin, Di- oder Triethylamin, Pyridin, Benzyl-trimethyl-ammoniumhydroxid, 1,5-Diazabicyclo[4.3.0]non-5-en (DBN) sowie 1,8-Diaza-bicyclo[5.4.0]undec-7-en (DBU) genannt.

Werden bei den vor- und nachstehend beschriebenen Umsetzungen beispielsweise Säuren verwendet, so kommen, sofern nicht abweichend definiert, beispielsweise anorganische Säuren, wie Mineralsäuren, z.B. Schwefelsäure, Phosporsäure oder Halogenwasserstoffsäuren, organische Carbonsäuren, wie Niederalkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder Sulfonsäuren, wie $C_1$-$C_7$-Alkan- oder gegebenenfalls substituierte Benzolsulfonsäure, z.B. Methan- oder p-Toluolsulfonsäure, in Betracht.

Variante a):

Die Umsetzung von Verbindungen der Formel IIa mit Verbindungen der Formel IIb ($Y_1$ = Di-$C_1$-$C_4$-Alkylamino, Carbamoyl, $C_1$-$C_7$-Alkylcarbamoyl, Di-$C_1$-$C_7$-alkylcarbamoyl oder $C_1$-$C_7$-Alkyl, $Y_2$ = Hydroxy bzw. $Y_1 + Y_2$ = Imino, $Y_3$ jeweils = Wasserstoff) bzw. der Tautomeren der Formeln IIc bzw. IId wird vorteilhaft in Gegenwart einer der vorstehend aufgeführten Basen, beispielsweise eines Alkalimetallalkoholats, z.B. Natriumethylat oder Kalium-tert-butylat, durchgeführt. In einer bevorzugten Ausführungsform wird als Reaktionsmedium Dimethylsulfoxid verwendet.

Bei der Reaktion mit Verbindungen der Formel IIc gelangt man bevorzugt zu solchen Verbindungen der Formel I, worin $R_1$ $C_1$-$C_7$-Alkyl bedeutet, während bei Verwendung von Verbindungen der Formel IId Verbindungen der Formel I erhalten werden, worin $R_1$ Amino bedeutet.

Die Ausgangsstoffe der Formel IIa können beispielsweise hergestellt werden durch Umsetzung einer Verbindung der Formel R-Hal (IIe; Hal = Halogen, z.B. Chlor, Brom oder Jod) mit Stickstoffwasserstoffsäure oder einem Salz davon, z.B. mit Natriumazid. Ausgangsstoffe der Formeln IIb, IIc und

IId sind bekannt.

Variante b):

Die solvolytische Ueberführung von $X_1$ bzw. $X_2$ in $R_1$ bzw. $R_2$ erfolgt in an sich bekannter Weise durch Hydrolyse, Ammonolyse oder Aminolyse mit einem $C_1$-$C_7$-Alkyl- oder Di-$C_1$-$C_7$-alkylamin. Die Solvolyse wird erforderlichenfalls in Gegenwart einer der vorstehend aufgeführten Säure oder Base, ferner gegebenenfalls mit Hilfe eines Dehydratisierungsmittels, durchgeführt werden.

Dehydratisierungsmittel sind beispielsweise Säureanhydride, wie Phosphorpentoxid, Acetylchlorid oder dergleichen, oder Carbodiimide, wie Dicyclohexylcarbodiimid.

So kann beispielsweise Cyano $X_1$ bzw. $X_2$ durch Hydrolyse in Carbamoyl übergeführt werden, während die Ammonolyse bzw. Aminolyse von verestertem oder anhydridisiertem Carboxy $X_1$ bzw. $X_2$ zu entsprechendem gegebenenfalls substituiertem Carbamoyl führt. Ferner kann durch Behandlung mit einem Dehydratisierungsmittel, wie Phosphorpentoxid, Ammoniumcarboxylat $X_1$ bzw. $X_2$ zu Carbamoyl dehydratisiert und die $C_1$-$C_7$-Alkoxy-iminomethylgruppe $X_1$ bzw. $X_2$, welche vorzugsweise durch Alkoholyse von Cyano in situ erhalten werden kann, unter den Reaktionsbedingungen oder durch Erwärmen in Carbamoyl bzw. $C_1$-$C_7$-Alkylcarbamoyl übergeführt werden. Ebenso kann die Ueberführung von Carboxy bzw. aktiviertem Carboxy in gegebenenfalls entsprechend substituiertes Carbamoyl durch Amidierung erfolgen. Die verfahrensgemässe Amidierung wird erforderlichenfalls in Gegenwart eines, insbesondere basischen, Kondensationsmittels ausgeführt, wobei als Basen in erster Linie die dem gewünschten gegebenenfalls substituierten Carbamoyl entsprechenden Amine bzw. Ammoniak, ferner die vorstehend genannten Basen unterstützend verwendet werden. Den Kondensationsmitteln zuzurechnen sind z.B. die bei der Bildung von Amidbindungen üblichen Dehydratisierungsmittel, die insbesondere verwendet werden, wenn $X_1$ bzw. $X_2$ Carboxy bedeuten. Dabei können beispielsweise in situ aktivierte Carboxyderivate, insbesondere aktivierte Ester bzw. Anhydride, z.B. der vorstehend aufgeführten Art, gebildet werden. Dehydratisierungsmittel sind beispielsweise Carbodiimide, z.B. N,N'-Diniederalkyl- oder N,N'-Dicycloalkyl-carbodiimid, wie N,N'-Diethyl-, N,N'-Diisopropyl- oder N,N'| Dicyclohexyl-carbodiimid, vorteilhaft unter Zusatz von N-Hydroxysuccinimid oder gegebenenfalls, z.B. durch Halogen, Niederalkyl oder Niederalkoxy, substituiertes 1-Hydroxy-benzotriazol oder N-Hydroxy-5-norbornen-2,3-dicarboxamid, N,N'-Diimidazolcarbonyl, eine geeignete Phosphoryl- bzw. Phosphinverbindung z.B. Diethylphosphonylcyanid, oder Diphenylphosphonylazid, ein 1-$C_1$-$C_7$-Alkyl-2-halogenpyridiniumhalogenid, z.B. 1-Methyl-2-chlor-pyridiniumiodid, oder 1,1'-(Carbonyldioxy)-bisbenzotriazol.

Die Freisetzung der Amino- bzw. $C_1$-$C_4$-Alkylaminogruppe erfolgt je nach Art der Aminoschutzgruppe in an sich bekannter Weise, z.B. durch Solvolyse, wie Hydrolyse, Acidolyse, durch Reduktion, z.B. durch Hydrogenolyse in Gegenwart eines Hydrierungskatalysators oder mittels eines Reduktionssystems aus Metall und protonenabspaltendem Mittel, wobei je nach Art der Schutzgruppen verschiedenartige (auch andersartige) sowie selektive Abspaltmethoden angewendet werden können.

So kann beispielsweise gegebenenfalls durch Halogen substituiertes $C_1$-$C_7$-Alkanoyl-, gegebenenfalls substituierte Benzoyl- oder $C_1$-$C_7$-Alkoxycarbonylreste durch Hydrolyse, vorzugsweise in Gegenwart einer Base und 1-$C_1$-$C_7$-Alkanoyl- bzw. 1-$C_1$-$C_7$-Alkoxycarbonyl-prop-1-en-2-ylreste durch säurekatalysierte Hydrolyse abgespalten werden. Entsprechende bevorzugte Reste sind beispielsweise Formyl, Acetyl 2-Brom-, 2-Jod-, 2,2,2-Trichloracetyl, Benzoyl, 4-Nitrobenzoyl, Ethoxycarbonyl, 1-Acetyl-prop-1-en-2-yl oder 1-Ethoxy-prop-1-en-2-yl.

Die Hydrolyse erfolgt in an sich bekannter Weise mit Hilfe von Wasser, wobei vorteilhaft in Gegenwart einer die Hydrolyse unterstützenden Säure oder Base, gegebenenfalls in Gegenwart eines inerten Lösungsmittels oder Verdünnungsmittels und/oder unter Kühlen oder Erwärmen gearbeitet wird.

Als Säuren eignen sich beispielsweise die vorstehend aufgeführten Protonsäuren, während als Basen die vorstehend aufgeführten, insbesondere Alkalimetall- oder Erdalkalimetallhydroxide bzw. -carbonate verwendet werden können, wie Natrium-, Kalium- oder Calciumhydroxid, Natrium- oder Kaliumcarbonat.

Durch Acidolyse spaltbare Aminoschutzgruppen sind beispielsweise $C_1$-$C_7$-Alkoxycarbonyl, Arylmethoxycarbonyl, Halogen-$C_2$-$C_7$-alkoxycarbonyl, 1-$C_1$-$C_7$-Alkanoyl- oder 1-$C_1$-$C_7$-Alkoxycarbonyl-prop-1-en-2-yl, in erster Linie tert-Butyloxy-, Diphenylmethoxycarbonyl, 1-Acetyl- oder 1-Ethoxycarbonyl-prop-1-en-2-yl.

Bei der acidolytischen Spaltung werden in der Regel z.B. starke Protonsäuren verwendet, wie Mineralsäuren, z.B. die Halogenwasserstoffsäuren Chlor-, Brom- oder Iodwasserstoffsäure, Perchlorsäure, gegebenenfalls geeignet substituierte $C_1$-$C_4$-Alkancarbonsäuren, wie Ameisensäure, Eisessig oder Trifluoressigsäure, Sulfonsäuren, wie gegebenenfalls substituierte Phenylsulfonsäuren, z.B. p-Bromphenyloder p-Toluolsulfonsäure, oder Gemische derselben, wie Bromwasserstoff/Eisessig-Gemische.

Als hydrogenolytisch spaltbare Aminoschutzgruppen kommen beispielsweise gegebenenfalls substituierte 1-Phenyl-$C_1$-$C_4$-alkoxy-carbonyl, Arylmethyl oder 2-$C_3$-$C_7$-Alkenyl, in erster Linie Benzyloxycarbonyl, Benzyl oder Allyl, in Betracht.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris(triphenylphosphin)-rhodium-I-halogenid, z.B. -chlorid, oder Raney-Nickel, die gegebenenfalls auf einem Trägermaterial, wie Aktivkohle, Alkalimetallcarbonat bzw. -sulfat oder einem Kieselgel, aufgezogen sind.

Als Aminoschutzgruppen, die mittels eines Reduktionssystems aus Metall und protonenabspaltendem Mittel abgespalten werden, sind beispielsweise 1-Phenyl-$C_1$-$C_4$-alkoxycarbonyl, Aroylmethoxycar-

bonyl oder 2-Halogen-$C_1$-$C_7$-alkoxycarbonyl, in erster Linie (4-Nitro-)-Benzyloxycarbonyl, 2-Iod- oder 2,2,2,-Trichlorethoxycarbonyl oder Phenacyloxycarbonyl, zu nennen.

Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Uebergangsmetall, z.B. Zink, Zinn, Eisen oder Titan, während als Protonen-abspaltendes Mittel, z.B. Protonsäuren der vorstehend genannten Art, wie Salz- oder Essigsäure, $C_1$-$C_7$-Alkanole, wie Ethanol, und/oder Amine bzw. Ammoniak in Frage kommen. Solche Systeme sind beispielsweise Natrium/Ammoniak, Zink/Salz- oder Essigsäure oder Zink/Ethanol.

Gegebenenfalls substituiertes 1-Phenyl-$C_1$-$C_4$-alkoxycarbonyl, insbesonders 4-Nitrobenzyloxycarbonyl, kann ferner z.B. mit einem Dithionit, wie Natriumdithionit, 1-Aroyl-methoxycarbonyl, insbesondere Phenacyloxycarbonyl, und 2-Halogen-$C_2$-$C_7$-alkanoyl z.B. mit Hilfe eines nucleophilen Reagens, wie einem Thiolat, z.B. Natrium thiophenolat, oder Thioharnstoff und Base und sich anschliessender Hydrolyse, und 2-$C_3$-$C_7$-Alkenyl, insbesondere Allyl oder But-2-enyl, mit Hilfe eines Rhodium(III)halogenids, wie Rhodium(III)chlorid, gespalten werden.

Das Ausgangsmaterial der Formel III kann z.B. in Analogie zur Variante a) hergestellt werden. So geht man beispielsweise von Verbindungen der Formel R-$N_3$ (IIa) aus und setzt diese mit einer Verbindung der Formel $X_1$-X-$X_2$ (IIIa), worin X die Gruppe -$CH_2$-CO-oder -C≡C- bedeutet, z.B. in Gegenwart einer Base, oder mit einer Verbindung der Formel NC-$CH_2$-$X_2$ (IIIb), z.B. in Gegenwart einer Base, um.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I kann in an sich bekannter Weise in eine andere Verbindung der Formel I umgewandelt werden.

Enthalten die Verbindungen der Formel (I) ungesättigte Reste R, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet sind, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80° bis etwa 200°C, vor allem zwischen Raumtemperatur und etwa 100°C durchgeführt werden. Die Reaktion erfolgt zweckmässig in einem Lösungsmittel, wie Wasser, einem $C_1$-$C_7$-Alkanol, z.B. Ethanol, Isopropanol oder n-Butanol, einem Ether, z.B. Dioxan, oder einer $C_1$-$C_7$-Alkancarbonsäure, z.B. Essigsäure.

Primäres Amino $R_1$ sowie primäres Amino als Bestandteil von Carbamoyl $R_1$ und/oder $R_2$ kann in Mono- oder Di-$C_1$-$C_7$-alkylamino übergeführt werden. Die $C_1$-$C_7$-Alkylierung erfolgt z.B. mit einem reaktiven Ester eines $C_1$-$C_7$-alkandiols wie einem $C_1$-$C_7$-Alkyl-halogenid, z.B. -bromid oder -iodid, $C_1$-$C_7$-alkylsulfonat, z.B. -methansulfonat oder -p-toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft eines Phasentransfer-Katalysators, wie Tetrabutylammoniumbromid oder Benzyltrimethylammoniumchlorid, wobei indes stärker basische Kondensationsmittel, wie Alkalimetallamide, -hydride oder -alkoholate, z.B. Natriumamid, Natriumhydrid oder Natriummethanolat, erforderlich sein können.

Die neuen Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronen ether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der

erfindungsgemässen Verbindungen entwickelt wurden, ihre Verwendung und Verfahren zu ihrer Herstellung bilden ebenfalls einen Gegenstand der Erfindung, wobei die Variablen R, $R_1$ und $R_2$ die für die jeweils bevorzugten Verbindungsgruppen der Formel I angegebenen Bedeutungen haben.

Die Erfindung betrifft ebenfalls die Verwendung der Verbindungen der Formel (I), insbesondere als pharmakologische, in erster Linie antikonvulsiv wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antikonvulsiva, z.B. zur Behandlung von Epilepsie, verwenden.

Die Erfindung betrifft gleichfalls pharmazeutische Präparate, die die erfindungsgemässen Verbindungen als Wirkstoffe enthalten, sowie Verfahren zu ihrer Herstellung.

Bei den erfindungsgemässen pharmazeutischen Präparaten, welche die erfindungsgemässe Verbindung enthalten, handelt es sich um solche zur enteralen, wie oralen ferner rektalen, und parenteralen sowie topischen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab.

Die neuen pharmazeutischen Präparate enthalten z.B. von etwa 10 % bis etwa 100 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferne Bindemittel, wie Stärkekleister, unter Verwendung von Mais-, Weizen-, Reis- oder Kartoffelstärke, Gelatine, Traganth, Methylcellulose und/oder Polyvinylpyrrolidon, wenn erwünscht, Sprengmittel, wie die obengenannten Stärken, ferner Carboxmethylstärke, quervernetzes Polyvinylpyrrolidon, Agar, Alginsäure oder ein Salz davon, wie Natriumalginat, Hilfsmittel sind in erster Linie Fliess-, Regulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Applikation eine ungefähre Tagesdosis von etwa 100 mg bis etwa 3000 mg, vorteilhaft in mehreren Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden und Druck in Pa angegeben.

Beispiel 1: Zu einer Mischung von 14,1 g (100 mM) 4-Azidoheptan, 12,6 g (150 mM) Cyanacetamid und 50 ml Dimethylsulfoxid gibt man eine warme Lösung

von 2,3 g (100 mM) Natrium in 50 ml absolutem Alkohol. Man rührt 20 Stunden bei Raumtemperatur, verdünnt mit 300 ml Eiswasser und neutralisiert mit konz. Salzsäure. Das ausgefallene Produkt wird abgesaugt und mehrmals mit Wasser gewaschen. Nach Umkristallisation aus Essigester-Toluol erhält man so 5-Amino-1-(4-heptyl)-1H-1,2,3-triazol-4-carboxamid vom Smp. 181-183°

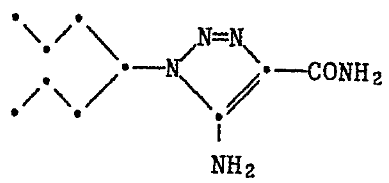

Beispiel 2: In analoger Weise wie in Beispiel 1 beschrieben erhält man unter Verwendung von Cyanessigsäure-monomethylamid das 5-Amino--1-(4-heptyl)-1H-1,2,3-triazol-4-carbonsäuremethylamid vom Smp. 151-153°.

Beispiel 3: In analoger Weise wie in Beispiel 1 beschrieben erhält man unter Verwendung von Cyanessigsäure-dimethylamid das 5-Amino-1-(4-hepty)-1H-1,2,3-triazol-4-carbonsäure-dimethylamid vom Smp. 91-92°.

Beispiel 4: In analoger Weise wie in Beispiel 1 beschrieben erhält man unter Verwendung von 1-Azidoheptan das 5-Amino-1-heptyl-1H-1,2,3-triazol-4-carboxamid vom Smp. 197-198°.

Beispiel 5: In analoger Weise wie in Beispiel 1 beschrieben erhält man unter Verwendung von 3-Azidopentan das 5-Amino-1-(3-pentyl)-1H-1,2,3-triazol-4-carboxamid vom Smp. 153-155°.

Beispiel 6: In analoger Weise wie in Beispiel 1 beschrieben erhält man unter Verwendung von 5-Azido-nonan das 5-Amino-1-(5-nonyl)-1H-1,2,3-triazol-4-carboxamid vom Smp. 169-171°.

Beispiel 7: In analoger Weise wie in Beispiel 1 beschrieben erhält man unter Verwendung von 4-Azido-heptadien-1,6 das 5-Amino-1-(hepta-1,6-dien-4-yl)-1H-1,2,3-triazol-4-carboxamid vom Smp. 140-142°.

Beispiel 8: In analoger Weise wie in Beispiel 1 beschrieben erhält man unter Verwendung von 2-Azidopropan das 5-Amino-1-(2-propyl)-1H-1,2,3-triazol-4-carboxamid vom Smp. 205-207°.

Beispiel 9: 8,5 g (60 mM) 4-Azido-heptan, 5,0 g (60mM) Tetrolsäureamid und 10 ml Dioxan werden bei Badtemperatur 130° 18 Stunden zum schwachen Rückfluss erhitzt. Nach Abkühlen und Verdünnen mit 50 ml Wasser saugt man das ausgefallene Produktgemisch ab.

Durch Chromatographie an Kieselgel mit Toluol-Essigester Gemischen erhält man aus der ersten Fraktion das 1-(4-Heptyl)5-methyl-1H-1,2,3-triazol-4-carboxamid vom Smp. 124-126°.

Beispiel 10: 225 g (1 Mol) 1-(4-Heptyl)-5-methyl-1H-1,2,3-triazol-4-carbonsäure, 500 ml Toluol, 262 g (2,2 Mol) Thionylchlorid und 1 ml Dimethylformamid werden 5 Stunden am Rückfluss gekocht und dann im Vakuum bei 70° vollständig eingedampft. Das rohe Säurechlorid löst man in 1 l Cyclohexan und tropft diese Lösung unter gutem Rühren zu 1 l eisgekühltem konzentriertem wässrigem Ammoniak. Das ausgefallene Produkt wird abgesaugt, mit Wasser gewaschen und nach dem Trocknen aus Benzol-Cyclohexan umkristallisiert. Man erhält so das 1-(4-Heptyl)-5-methyl-1H-1,2,3-triazol-4-carboxamid vom Smp. 124-126°.

Das Ausgangsmaterial kann wie folgt hergestellt werden: Zu einer Lösung von 37 g (1,6 Mol) Natrium in 1 l abs. Alkohol gibt man eine Mischung von 208 g (1,6 Mol) Acetessigsäureethylester und 113 g (0,8 Mol) 4-Azido-heptan und kocht während 48 Stunden am Rückfluss. Dann tropft man 0,8 l 2n wässrige Natronlauge zu und hält weitere 2 Stunden am Rückfluss. Nach Abdestillation von 1 l Alkohol verdünnt man den Rückstand mit 1,5 l Wasser, klärt die Lösung durch Filtration mit Entfärbungskohle und stellt mit 0,4 l konzentrierter Salzsäure sauer. Das ausgefallene Produkt wird abgesaugt und mehrmals mit Wasser gewaschen. Nach dem Trocknen bei 80° erhält man so die 1-(4-Heptyl)-5-methyl-1H-1,2,3-triazol-4-carbonsäure vom Smp. 129-131°.

Beispiel 11: 11,3 g (40 mM) 1-(4-Heptyl)-1H-1,2,3-triazol-4,5-dicarbonsäure-dimethylester löst man in 200 ml 4n-methanolischem Ammoniak und heizt im verschlossenen Gefäss 16 Stunden auf 70°. Nach Abdampfen des Lösungsmittels kristallisiert man den Rückstand aus Benzol-Petrolether und erhält so das 1-(4-Heptyl)-1H-1,2,3-triazol-4,5-biscarboxamid vom Smp. 133-135°.

Das Ausgangsmaterial kann wie folgt hergestellt werden: 7,05 g (50 mM) 4-Azidoheptan und 7,1 g (50 mM) Acetylendicarbonsäuredimethylester werden in 100 ml Benzol 8 Stunden am Rückfluss gekocht und nach dem Abkühlen auf eine Säule mit 150 g Kieselgel aufgezogen. Man eluiert mit Benzol-Essigester 4:1 und erhält so den 1-(4-Heptyl)-1H-1,2,3-triazol-4,5-dicarbonsäure-dimethylester als farblose Flüssigkeit. Er wird ohne weitere Reinigung in der Ammonolyse eingesetzt.

Beispiel 12: 12 g (50 mM) 5-Amino-1-(4-heptyl)-1H-1,2,3-triazol-4-carbonsäuremethylamid löst man in gemischtem Anhydrid aus 35 ml Acetanhydrid und 35 ml wasserfreier Ameisensäure und lässt 15 Stunden bei Raumtemperatur rühren. Nach Abdampfen der flüchtigen Teile löst man den Rückstand in 130 ml In-Natronlauge und tropft bei Raumtemperatur während 30 Minuten 8,1 g (64 mM) Dimethylsulfat zu. Man rührt noch 1 Stunde nach und saugt das ausgefallene Produkt ab. Nach Umkristallisation aus verdünntem Ethanol erhält man das 1-(4-Heptyl)-5-methylamino-1H-1,2,3-triazol-4-carbonsäuremethylamid vom Smp. 88-90°.

Beispiel 13: Tabletten, enthaltend je 50 mg des Wirkstoffs, z.B. 5- Amino-1-(4-heptyl)-1H-1,2,3-triazol-4-carboxamid, können wie folgt hergestellt werden.

Zusammensetzung (10000 Tabletten)

Wirkstoff 500.0 g
Lactose 500.0 g
Kartoffelstärke 352.0 g
Gelatine 8.0 g

Talk 60.0 g
Magnesiumstearat 10.0 g
Siliciumdioxid (hochdisper.) 20.0 g
Ethanol q.s.

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 14: Lacktabletten, enthaltend je 100 mg des Wirkstoff, z.B. 5-Amino-1-(4-heptyl)-1H-1,2,3-triazol-4-carboxamid, können wie folgt hergestellt werden:

Zusammensetzung (für 1000 Tabletten)
Wirkstoff 100.00 g
Lactose 100.00 g
Maisstärke 70.00 g
Talk 8.50 g
Calciumstearat 1.50 g
Hydroxypropyl-methylcellulsoe 2.36 g
Schellack 0.64 g
Wasser q.s.
Methylenchlorid q.s.

Der Wirkstoff, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 15: In analoger Weise wie in den Beispielen 13 und 14 beschrieben können auch Tabletten bzw. Lacktabletten enthaltend eine andere Verbindung gemäss der Beispiele 1-12 hergestellt werden.

**Patentansprüche**

1. Trisubstituierte 1,2,3-Triazole der Formel

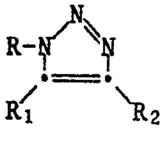 (I),

worin R einen aliphatischen Rest bedeutet, $R_1$ Amino, $C_1$-$C_4$-Alkylamino, Di-$C_1$-$C_4$-Alkylamino, Carbamoyl, $C_1$-$C_7$-Alkylcarbamoyl, Di-$C_1$-$C_7$-alkyl-carbamoyl oder $C_1$-$C_7$-Alkyl bedeutet und $R_2$ Carbamoyl, $C_1$-$C_7$-Alkylcarbamoyl oder Di-$C_1$-$C_7$-alkyl-carbamoyl bedeutet.

2. Verbindung gemäss Anspruch 1 der Formel I, worin R über ein sekundäres C-Atom gebundenes $C_5$-$C_{10}$-Alkyl bzw. $C_5$-$C_{10}$-Alkenyl, welches eine oder zwei Doppelbindungen aufweist, bedeutet, $R_1$ Amino, $C_1$-$C_4$-Alkylamino, Di-$C_1$-$C_4$-Alkylamino, Carbamoyl, $C_1$-$C_4$-Alkylcarbamoyl, Di-$C_1$-$C_4$-alkyl-carbamoyl oder $C_1$-$C_4$-Alkyl bedeutet und $R_2$ Carbamoyl, $C_1$-$C_4$-Alkylcarbamoyl oder Di-$C_1$-$C_4$-alkyl-carbamoyl bedeutet.

3. Verbindung gemäss Anspruch 1 der Formel I, worin R über ein sekundäres C-Atom gebundenes $C_5$-$C_{10}$-Alkyl bzw. $C_5$-$C_{10}$-Alkenyl, welches eine oder zwei Doppelbindungen aufweist, bedeutet, $R_1$ Amino, Carbamoyl, $C_1$-$C_4$-Alkylcarbamoyl, Di-$C_1$-$C_4$-alkyl-carbamoyl oder $C_1$-$C_4$-Alkyl bedeutet und $R_2$ Carbamoyl, $C_1$-$C_4$-Alkylcarbamoyl oder Di-$C_1$-$C_4$-alkyl-carbamoyl bedeutet.

4. Verbindung gemäss Anspruch 1 der Formel I, worin R über ein sekundäres C-Atom gebundenes $C_5$-$C_8$-Alkyl bzw. $C_5$-$C_8$-Alkenyl mit einer oder zwei Doppelbindungen bedeutet, $R_1$ Amino, Carbamoyl oder $C_1$-/$C_4$-Alkyl bedeutet und $R_2$ Carbamoyl, $C_1$-$C_4$-Alkylcarbamoyl oder Di-$C_1$-$C_4$-alkyl-carbamoyl bedeutet.

5. Verbindung gemäss Anspruch 1 der Formel I, worin R 1-n-Propyl-n-butyl oder 1-Allyl-3-butenyl bedeutet, $R_1$ Amino, Carbamoyl oder $C_1$-$C_4$-Alkyl, insbesondere Methyl, bedeutet und $R_2$ Carbamoyl ist.

6. Verbindungen gemäss einem der Ansprüche 1 bis 5, worin $R_1$ Amino und $R_2$ Carbamoyl ist.

7. 5-Amino-1-(4-heptyl)-1H-1,2,3-triazol-4-carboxamid gemäss Anspruch 1.

8. 5-Amino-1-(4-heptyl)-1H-1,2,3-triazol-4-carbonsäure-methylamid, 5-Amino-1-(4-heptyl)-1H-1,2,3-triazol-4-carbonsäure-dimethylamid, 5-Amino-1-heptyl-1H-1,2,3-triazol-4-carboxamid, 5-Amino-1-(3-pentyl)-1H-1,2,3-triazol-4-carboxamid, 5-Amino-1-(5-nonyl)-1H-1,2,3-triazol-4-carboxamid, 5-Amino--1-(hepta-1,6-dien-4-yl)-1H-1,2,3-triazol-4-carboxamid, 5-Amino-1-(2-propyl)-1H-1,2,3-triazol-4-carboxamid, 1-(4-Heptyl)-5-methyl-1H-1,2,3-triazol-4-carboxamid, 1-(4-Heptyl)-1H-1,2,3-triazol-4,5-biscarboxamid oder 1-(4-Heptyl)-5-methylamino-1H-1,2,3-triazol-4-carbonsäure-methylamid gemäss Anspruch 1.

9. Verbindung gemäss einem der Ansprüche 3 - 5, 7 und 8 zur Anwendung in einem Verfahren zur therapeutischen oder prophylaktischen Behandlung des menschlichen oder tierischen Körpers, z.B. zur Verwendung als Antikonvulsivum.

10. Verbindungen gemäss Anspruch 1, 2 und 6 zur Anwendung in einem Verfahren zur therapeutischen oder prophylaktischen Behandlung des menschlichen oder tierischen Körpers, z.B. zur Verwendung als Antikonvulsivum.

11. Pharmazeutisch Präparate, enthaltend eine Verbindung gemäss einem der Ansprüche 3 - 5 und 7 - 9.

12. Pharmazeutische Präparate, enthaltend eine Verbindung gemäss einem der Ansprüche 1, 2, 6 und 10.

13. Verwendung von Verbindungen gemäss einem der Ansprüche 1 - 10 zur Herstellung von Antikonvulsiva.

14. Verfahren zur Herstellung von Verbindungen der Formel I gemäss einem der Ansprüche 1 - 8, dadurch gekennzeichnet, dass man

a) eine Verbindung der Formel R-$N_3$ (IIa) mit einer Verbindung der Formel

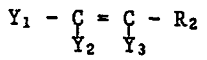 (IIb)

worin $Y_1$ $D_1$-$C_1$-$C_4$-Alkylamino, Carbamoyl, $C_1$-$C_7$-Alkylcarbamoyl, $D_1$-$C_1$-$C_7$-Alkylcarbamoyl oder $C_1$-$C_7$-Alkyl bedeutet und $Y_2$ Hydroxy und $Y_3$ Wasserstoff bedeutet oder $Y_2$ und $Y_3$ gemeinsam eine zusätzliche Bindung darstellen, oder worin $Y_1$ und $Y_2$ gemeinsam Imino darstellen und $Y_3$ Wasserstoff ist, oder einem Tautomeren und/oder Salz davon umsetzt oder

b) in einer Verbindung der Formel

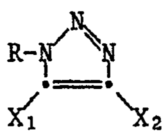 (III)

oder einem Salz davon, worin $X_1$ einen in $R_1$ überführbaren Rest bedeutet und $X_2$ einen in $R_2$ überführbaren Rest oder $R_2$ bedeutet oder worin $X_1$ für $R_1$ steht und $X_2$ einen in $R_2$ überführbaren Rest bedeutet, $X_1$ und/oder $X_2$ in $R_1$ bzw. $R_2$ überführt und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt.

Patentansprüche für folgende Vertragsstaaten: AT, GR, ES

1. Verfahren zur Herstellung von trisubstituierten 1,2,3-Triazolen der Formel

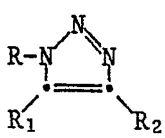 (I),

worin R einen aliphatischen Rest bedeutet, $R_1$ Amino, $C_1$-$C_4$-Alkylamino, Di-$C_1$-$C_4$-Alkylamino, Carbamoyl, $C_1$-$C_7$-Alkylcarbamoyl, Di-$C_1$-$C_7$-alkyl-carbamoyl oder $C_1$-$C_7$-Alkyl bedeutet und $R_2$ Carbamoyl, $C_1$-$C_7$-Alkylcarbamoyl oder Di-$C_1$-$C_7$-alkyl-bedeutet, dadurch gekennzeichnet, dass man

a) eine Verbindung der Formel R-$N_3$ (IIa) mit einer Verbindung der Formel

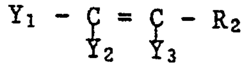 (IIb)

worin $Y_1$ $D_1$-$C_1$-$C_4$-Alkylamino, Carbamoyl, $C_1$-$C_7$-Alkylcarbamoyl, $D_1$-$C_1$-$C_7$-Alkylcarbamoyl oder $C_1$-$C_7$-Alkyl bedeutet und $Y_2$ Hydroxy und $Y_3$ Wasserstoff bedeutet oder $Y_2$ und $Y_3$ gemeinsam eine zusätzliche Bindung darstellen, oder worin $Y_1$ und $Y_2$ gemeinsam Imino darstellen und $Y_3$ Wasserstoff ist, oder einem Tautomeren und/oder Salz davon umsetzt oder

b) in einer Verbindung der Formel

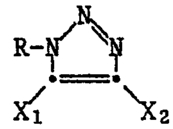 (III)

oder einem Salz davon, worin $X_1$ einen in $R_1$ überführbaren Rest bedeutet und $X_2$ einen in $R_2$ überführbaren Rest oder $R_2$ bedeutet oder worin $X_1$ für $R_1$ steht und $X_2$ einen in $R_2$ überführbaren Rest bedeu tet, $X_1$ und/oder $X_2$ in $R_1$ bzw. $R_2$ überführt und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt.

2. Verfahren gemäss Anspruch 1, dadurch gekennzeichnet, dass man einen Ausgangsstoff der Formel IIb, worin $Y_1$ Carbamoyl, $C_1$-$C_7$-Alkylcarbamoyl, Di-$C_1$-$C_4$-Alkylcarbamoyl oder $C_1$-$C_7$-Alkyl und $Y_2$ Hydroxy bedeutet bzw. $Y_1$ und $Y_2$ gemeinsam Imino darstellen und $Y_3$ jeweils Wasserstoff bedeutet, in der tautomeren Keto- bzw. Nitrilform der Formeln $Y_1$-C(=O)-$CH_2$-$R_2$ (IIC) bzw. N≡C-$CH_2$-$R_2$ (IId) einsetzt.

3. Verfahren gemäss Anspruch 1 oder 2 zur Herstellung von Verbindung der Formel I, worin R über ein sekundäres C-Atom gebundenes $C_5$-$C_{10}$-Alkyl bzw. $C_5$-$C_{10}$-Alkenyl, welches eine oder zwei Doppelbindungen aufweist, bedeutet, $R_1$ Amino, $C_1$-$C_4$-Alkylamino, Di-$C_1$-$C_4$-Alkylamino Carbamoyl, $C_1$-$C_4$-Alkylcarbamoyl, Di-$C_1$-$C_4$-alkyl-carbamoyl oder $C_1$-$C_4$-Alkyl bedeutet und $R_2$ Carbamoyl, $C_1$-$C_4$-Alkylcarbamoyl oder Di-$C_1$-$C_4$-alkyl-carbamoyl bedeutet.

4. Verfahren gemäss Anspruch 1 oder 2 zur Herstellung von Verbindung der Formel I, worin R über ein sekundäres C-Atom gebundenes $C_5$-$C_{10}$-Alkyl bzw. $C_5$-$C_{10}$-Alkenyl, welches eine oder zwei Doppelbindungen aufweist, bedeutet, $R_1$ Amino, Carbamoyl, $C_1$-$C_4$-Alkylcarbamoyl, Di-$C_1$-$C_4$-alkyl-carbamoyl oder $C_1$-$C_4$-Alkyl bedeutet und $R_2$ Carbamoyl, $C_1$-$C_4$-Alkylcarbamoyl oder Di-$C_1$-$C_4$-alkyl-carbamoyl bedeutet.

5. Verfahren gemäss Anspruch 1 oder 2 zur Herstellung von Verbindung der Formel I, worin R über ein sekundäres C-Atom gebundenes $C_5$-$C_8$-Alkyl bzw. $C_5$-$C_8$-Alkenyl mit einer oder zwei Doppelbindungen bedeutet, $R_1$ Amino,

Carbamoyl oder $C_1$-$C_4$-Alkyl bedeutet und $R_2$ Carbamoyl, $C_1$-$C_4$-Alkylcarbamoyl oder Di-$C_1$-$C_4$-alkyl-carbamoyl bedeutet.

6. Verfahren gemäss Anspruch 1 oder 2 zur Herstellung von Verbindung der Formel I, worin R 1-n-Propyl-n-butyl oder 1-Allyl-3-butenyl bedeutet, $R_1$ Amino, Carbamoyl oder $C_1$-$C_4$-Alkyl, insbesondere Methyl, bedeutet und $R_2$ Carbamoyl ist.

7. Verfahren gemäss einem der Ansprüche 1 bis 6 zur Herstellung von Verbindung der Formel I, worin $R_1$ Amino und $R_2$ Carbamoyl ist.

8. Verfahren gemäss Anspruch 1 oder 2 zur Herstellung von 5-Amino-1-(4-heptyl)-1H-1,2,3-triazol-4-carboxamid.

9. Verfahren gemäss Anspruch 1 oder 2 zur Herstellung von 5-Amino-1-(4-heptyl)-1H-1,2,3-triazol-4-carbonsäuremethylamid, 5-Amino-1-(4-heptyl)-1H-1,2,3-triazol-4-carbonsäure-dimethylamid, 5-Amino-1-heptyl-1H-1,2,3-triazol-4-carboxamid, 5-Amino-1-(3-pentyl)-1H-1,2,3-triazol-4-carboxamid, 5-Amino-1-(5-nonyl)-1H-1,2,3-triazol-4-carboxamid, 5-Amino-1-(hepta-1,6-4-yl)-1H-1,2,3-triazol-4-carboxamid, 5-Amino-1-(2-propyl)-1H-1,2,3-triazol-4-carboxamid, 1-(4-Heptyl)-5-methyl-1H-1,2,3-triazol-4-carboxamid, 1-(4-Heptyl)-1H-1,2,3-triazol-4,5-biscarboxamid oder 1-(4-Heptyl)-5-methylamino-1H-1,2,3-triazol-4-carbonsäuremethylamid.

10. Verfahren zur Herstellung pharmazeutischer Präparate, enthaltend eine Verbindung gemäss einem der Ansprüche 1 - 9, dadurch gekennzeichnet, dass man eine gemäss einem der Ansprüche 1 - 8 erhältliche Verbindung gegebenenfalls unter Beimischung von üblichen Hilfs- oder Trägerstoffen, zu pharmazeutischen Präparaten verarbeitet.

11. Verfahren gemäss Anspruch 10 zur Herstellung von Antikonvulsiva.

Europäisches Patentamt

# EUROPÄISCHER RECHERCHENBERICHT

Nummer der Anmeldung

Ep 87100012.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | CHEMICAL ABSTRACTS, Band 91, Nr. 21, 19. November 1979, Columbus, Ohio, USA<br>ALBERT, ADRIEN; TROTTER,A.MARK "v-Triazolo[4,5-d]pyrimidines (8-azapurines). Part. 21. Synthesis of 2-substituted 8-azapurin-6-ones from 4-amino-1,2,3-triazole-5-carboxamides and amidines."<br>Seite 659, Spalte 1, Zusammenfassung Nr. 175 286d<br>& J.Chem.Soc.,Perkin Trans. 1 1979, (4), 922-5<br>-- | 1 | C 07 D 249/04<br>A 61 K 31/41 |
| A | CHEMICAL ABSTRACTS, Band 101, Nr. 17, 22. Oktober 1984, Columbus, Ohio, USA<br>FAN, RULIN "Synthesis of some 1,2,3-triazole derivatives."<br>Seite 712, Spalte 2, Zusammenfassung Nr. 151 802t<br>& Huadong Huagong Xueyuan Xuebao 1984, (1), 121-5 (Ch.)<br>-- | 1 | RECHERCHIERTE SACHGEBIETE (Int. Cl. 4)<br>C 07 D 249/00 |
| X | DE - A1 - 2 856 873 (SANDOZ)<br>* Formel II; Beispiel 99 *<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 20-03-1987 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung: EP 87100012.1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | CHEMICAL ABSTRACTS, Band 101, Nr. 25, 17. Dezember 1984, Columbus, Ohio, USA<br>CIBA-GEIGY A.-G. "Aralkyltriazoles" Seite 773, Spalte 1, Zusammenfassung Nr. 230 539r<br>& Jpn. Kokai Tokkyo Koho JP 59,118,775[84,118,775]<br>-- | 1 | |
| A | CHEMICAL ABSTRACTS, Band 95, Nr. 17, 26. Oktober 1981, Columbus, Ohio, USA<br>ALBERT, ADRIEN "v-Triazolo[4,5-d]--pyrimidines (8-azapurines). Part 24. The 3-alkyl derivatives." Seite 665, Spalte 1, Zusammenfassung Nr. 150 600w<br>& J.Chem.Soc.,Perkin Trans. 1 1981, (8), 2344-51<br>-- | 1 | RECHERCHIERTE SACHGEBIETE (Int. Cl.4) |
| A | CHEMICAL ABSTRACTS, Band 91, Nr. 25, 17. Dezember 1979, Columbus, Ohio, USA<br>LUCACCHINI,A.; SEGNINI,D.; DA SETTIMO, A.; LIVI, O. "Effects of substituted 1,2,3-triazoles on adenosine deaminase, guanine deaminase and xanthine oxidase." Seite 27, Spalte 2, Zusammenfassung Nr. 204 219s<br>& Ital.J.Biochem. 1979, 28(3), 194-206 (Eng).<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 20-03-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 87100012.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | EP - A2 - 0 151 528 (MERCK)<br>* Zusammenfassung *<br>----- | 1 | |
| | | | RECHERCHIERTE SACHGEBIETE (Int. Cl 4) |

Der vorliegende Recherchenbericht wurde für alle Patentansprüche erstellt

| Recherchenort | Abschlußdatum der Recherche | Prüfer |
|---|---|---|
| WIEN | 20-03-1987 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82